(19) Europäisches Patentamt
European Patent Office
Office européen des brevets

(11) Veröffentlichungsnummer: **0 081 748**
**B1**

(12) # EUROPÄISCHE PATENTSCHRIFT

(45) Veröffentlichungstag der Patentschrift: **25.02.87**

(51) Int. Cl.⁴: **C 07 D 233/22**, A 61 K 31/415

(21) Anmeldenummer: **82111098.8**

(22) Anmeldetag: **01.12.82**

(54) **Verfahren zur Herstellung von (-)-2-(1-(2,6-Dichlorphenoxy)-ethyl)-1,3-diazacyclopent-2-en.**

(30) Priorität: **10.12.81 DE 3149009**

(43) Veröffentlichungstag der Anmeldung: **22.06.83 Patentblatt 83/25**

(45) Bekanntmachung des Hinweises auf die Patenterteilung: **25.02.87 Patentblatt 87/9**

(84) Benannte Vertragsstaaten: **AT BE CH DE FR GB IT LI LU NL SE**

(56) Entgegenhaltungen:
**DE-A-1 695 555**
**DE-A-1 795 843**
**DE-A-1 935 479**
**DE-A-2 818 367**

**JERRY MARCH: "Advanced organic chemistry: reactions, mechanisms, and structure", International Student Edition, 1968, McGraw-Hill Book Company, New York, US**
**Pharmazie in unserer Zeit, 2,(1973), 73-77**
**Deutsche Apotheker-Zeitung, 116 Jahrg., Nr. 24, 849-851; Nr. 14, 685-691**

(73) Patentinhaber: **A. Nattermann & Cie. GmbH, Nattermannallee 1, D-5000 Köln 30 (DE)**

(72) Erfinder: **Biedermann, Jürgen, Dr., Kirschenweg 14, D-5024 Pulheim- Stommeln (DE)**
Erfinder: **Prop, Gerrit, Dr., Mohnblumenweg 25, D-5024 Pulheim (DE)**
Erfinder: **Doppelfeld, Ille- Stephanie, Im Bruchfeldchen 35, D-5151 Bergheim- Glessen (DE)**

(74) Vertreter: **Redies, Bernd, Dr. rer. nat., COHAUSZ & FLORACK Patentanwaltsbüro Schumannstrasse 97 Postfach 14 01 47, D-4000 Düsseldorf 1 (DE)**

**EP 0 081 748 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

**Beschreibung**

Die Erfindung betrifft ein Verfahren zur Herstellung von (-)-2-[1-(2,6-Dichlorphenoxy)-ethyl]-1,3-diazacyclopent-2-en der Formel

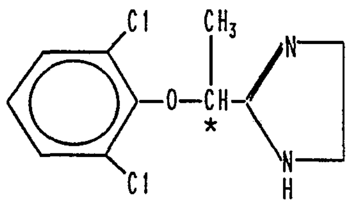

* Asymmetriezentrum

und seiner pharmazeutisch verträglichen Säureadditionssalze.

Herstellungsverfahren des Racemats (±)-2-[1-(2,6-Dichlorphenoxy)-ethyl]-1,3-diazacyclopent-2-en, das als Antihypertonikum bekannt ist, enthalten die Offenlegungsschriften DE–OS–16 95 555 und DE–OS 19 35 479 sowie die Patentschrift DE–PS 17 95 843.

Obwohl es bekannt war, daß 2-[1-(2,6-Dichlorphenoxy)-ethyl]-1,3-diazacyclopent-2-en wegen des asymmetrisch substituierten Kohlenstoffatoms in zwei optisch aktiven enantiomeren Formen vorliegen kann, wurde eine Trennung bzw. Herstellung der optisch aktiven links- bzw. rechtsdrehenden Enantiomeren bisher nicht durchgeführt.

Die Abtrennung des linksdrehenden Enantiomeren zur Aufklärung der Zusammenhänge zwischen optischer Aktivität und biologischer Wirksamkeit gelang durch die an sich bekannte Racematspaltungs-Methode mit Hilfe der linksdrehenden Dibenzoylweinsäure.

Die pharmakologischen Untersuchungen ergaben überraschenderweise, daß nur das linksdrehende Enantiomere eine stark ausgeprägte, langanhaltende Blutdrucksenkung hervorruft, während das rechtsdrehende Enantiomere in diesem Modell praktisch keine Wirkung zeigt.

Auch im anschließenden Vergleich der pharmakologischen Wirksamkeit des linksdrehenden Enantiomeren mit dem Racemat zeigte sich, daß die halbe Menge des linksdrehenden Enantiomeren ausreicht, um eine Blutdrucksenkung bestimmter Wirkungstiefe und Wirkungsdauer zu erreichen.

Die Synthese der erfindungsgemäßen optisch aktiven Verbindung ist dadurch gekennzeichnet, daß sie von optisch aktiven Ausgangssubstanzen ausgeht. So wird z. B. ein (-)-2-Hydroxypropionsäure-$C_{1-4}$-Alkylester mit überschüssigem Thionylchlorid in Gegenwart katalytischer Mengen von Dimethylformamid unter Konfigurationsumkehr zum (+)-2-Chlorpropionsäure-$C_{1-4}$-Alkylester umgesetzt. Die anschliessende Veretherung mit 2,6-Dichlorphenol erfolgt unter erneuter Konfigurationsumkehr in geeigneten organischen Lösungsmitteln, wie z. B. Acetonitril, Butanon, Dimethylformamid in Gegenwart von Hilfsbasen, wie z. B. Natriumhydrid, Alkalimethylat, Alkaliethylat oder 1,4-Diazabicyclo-(2,2,2)-octan im Temperaturbereich von 60 – 150°C, vorzugsweise bei 80°C zum $C_{1-4}$-Alkylester der (-)-2-(2,6-Dichlorphenoxy)-propionsäure, die neben ihren funktionellen Säurederivaten als Ausgangssubstanz für die Synthese von 1,3-Diazacyclopenten-Derivaten verwendet wird. Dabei kann die Umsetzung mit Ethylendiamin selbst oder mit einem reaktiven N-Derivat des Ethylendiamins oder mit Ammoniak bzw. mit ammoniakabgebenden Mitteln und einer in das Ethylendiamin durch Behandlung mit Ammoniak überführbaren Verbindung durchgeführt werden.

Als funktionelle Säurederivate können z. B. $C_{1-4}$-Alkylester, Säurehalogenide, Amide, Thiamide, Amidine, Imidsäureester oder das Nitril der 2-(2,6-Dichlorphenoxy)-propionsäure verwendet werden.

Im erfindungsgemäßen Verfahren wird (-)-2-(2,6-Dichlorphenoxy)-propionsäureethylester mit einem großen Überschuß an 1,2-Diaminoethan bei Raumtemperatur zum (-)-2-(2,6-Dichlorphenoxy)-propionsäure-N-(2-aminoethyl)-amid umgesetzt, das seinerseits mit einem Titantetrachlorid/Tetrahydrofuran-Komplex in Chloroform in Gegenwart von 4-Dimethylaminopyridin bei 0–30°C zum (-)-2-[1-(2,6-Dichlorphenoxy)-ethyl]-1,3-diazacyclopent-2-en dehydratisiert bzw. cyclisiert wird.

Die anschließende Überführung der linksdrehenden Base in ein physiologisch unbedenkliches Säureadditionssalz erfolgt mit einer geeigneten anorganischen oder organischen Säure in einem niederen Alkohol. So erhält man aus der (-)-Base z. B. mit Propanol-2/Chlorwasserstoff das (-)-2-[1-(2,6-Dichlorphenoxy)-ethyl]-1,3-diazacyclopenten-2-hydrochlorid.

Ein anderes Verfahren geht ebenfalls von (-)-2-(2,6-Dichlorphenoxy)-propionsäureethylester aus, der bei Raumtemperatur mit einer ethanolischen Lösung von Ammoniak zum in Ethanol schwerlöslichen (+)-2-(2,6-Dichlorphenoxy)-propionsäureamid umgesetzt wird. Dieses Amid wird bei 0-30°C mit einem Titantetrachlorid/Tetrahydrofuran-Komplex und 4-Methylmorpholin in Chloroform zum (-)-2-(2,6-Dichlorphenoxy)-propionsäurenitril dehydratisiert. Man überführt sodann das Nitril mit Ethanol in Chloroform durch Einleiten von Chlorwasserstoff bei 0°C in das (-)-2-(2,6-Dichlorphenoxy)-propionimidsäureethylester-hydrochlorid, löst dieses in Ethanol und cyclisiert durch Zugabe von 1,2-Diaminoethan zum (-)-2-[1-(2,6-Dichlorphenoxy)-ethyl]-1,3-diazacyclopent-2-en. Von der so erhaltenen (-)-Base wird analog dem vorangehenden Verfahren das (-)-2-[1-(2,6-Dichlorphenoxy)-ethyl]-1,3-diazacyclopenten-2-hydrochlorid hergestellt.

Die Racematspaltung des (±)-2-[1-(2,6-Dichlorphenoxy)-ethyl]-1,3-diazacyclopent-2-ens erfolgt in an sich bekannter Weise nach den in der organischen Chemie üblichen Verfahren (Racematspaltung: vergl. Übersicht in: Houben-Weyl, Methoden der organischen Chemie, Bd. 4/2, 505 (1955), Georg Thieme Verlag, Stuttgart). Man überführt die racemische Base in einem geeigneten Lösungsmittel, wie z. B. Aceton, mit der optisch aktiven (-)-Dibenzoylweinsäure in zwei durch ihre Löslichkeit von einander unterschiedliche Diastereomeren-Salzpaare. Das wegen seiner geringen Löslichkeit in Aceton auskristallisierte Salz, in dem die (-)-Base (-)-2-[1-(2,6-Dichlorphenoxy)-ethyl]-1,3-diazacyclopent-2-en stark angereichert als (-)-Dibenzoyltartrat vorliegt, wird abgesaugt, die (-)-Base mit gesättigter Natriumcarbonatlösung freigesetzt und erneut mit (-)-Dibenzoylweinsäure in Aceton versetzt. Das auf diese Weise erhaltene 2. Kristallisat liegt als (-)-Dibenzoyltartrat der mittlerweile hochangereicherten (-)-Base vor und wird durch zweimalige Kristallisation aus Ethanol weiter gereinigt. Mit Natriumcarbonatlösung wird die (-) -Base freigesetzt und mit Propanol-(2)/Chlorwasserstoff in das optisch reine (-)-2-[1-(2,6-Dichlorphenoxy)-ethyl-1,3-diazacyclopenten-2-hydrochlorid überführt.

Aus der freien Base der erfindungsgemäßen Verbindung können durch Behandlung mit geeigneten Säuren die verschiedensten pharmazeutisch verträglichen Säureadditionssalze nach üblichen Methoden hergestellt werden. Für die Herstellung derartiger Salze kommen anorganische Säuren, z. B. Bromwasserstoffsäure, Schwefelsäure oder Phosphorsäure, oder organische Säuren, wie z. B. Essigsäure, Glykolsäure, Bern-steinsäure, Fumarsäure, Äpfelsäure, Weinsäure, Citronensäure, Benzoesäure oder Zimtsäure in Frage.

Die erfindungsgemäße Verbindung und ihre pharmazeutisch verträglichen Säureadditionssalze weisen wertvolle pharmakologische Eigenschaften auf, insbesondere eine stark blutdrucksenkende Wirkung bei langer Wirkungsdauer und geringen Nebenwirkungen. Die Verminderung des peripheren Widerstandes ist zur Entlastung des Herzens bei Bluthochdruck erwünscht und macht die erfindungsgemäßen Verbindungen zur Behandlung von Kreislauferkrankungen besonders geeignet.

Die Herstellung der erfindungsgemäßen Verbindungen wird durch die folgenden Beispiele näher erläutert. Die angegebenen Schmelzpunkte wurden mit einem Büchi 510-Schmelzpunktbestimmungsapparat gemessen und sind in °C angegeben und nicht korrigiert.

**Beispiel 1**

Synthese von (-)-2-[1-(2,6-Dichlorphenoxy)-ethyl]-1,3-diazacyclopenten-(2)-hydrochlorid

(+)-2-Chlorpropionsäureethylester.

250 g (= 2,119 Mol) (-)-2-Hydroxypropionsäureethylester $[\alpha]_D^{20}$ = –11,0° (unverdünnt) werden mit 264,0 g (= 2,218 Mol) Thionylchlorid in Gegenwart von 1,5 ml Dimethylformamid in der Siedehitze chloriert. Man erhält 93,0 g (+)-2-Chlorpropionsäureethylester, $C_5H_9ClO_2$ [136,6] Kp: 143°C;

$[\alpha]_D^{20}$ = +19,8° (unverdünnt)

(-)-2-(2,6-Dichlorphenoxy)-propionsäureethylester.

65,5 g (= 0,402 Mol) 2,6-Dichlorphenol werden in 300 ml Butanon mit 38,0 g (= 0,542 Mol) Kaliummethylat und 76,0 g (= 0,556 Mol) (+)-2-Chlorpropionsäureethylester 48 Stunden unter Rühren und Rückfluß zum Sieden erhitzt. Nach Aufarbeitung erhält man 62,0 g (-)-2-(2,6-Dichlorphenoxy)-propionsäureethylester, $C_{11}H_{12}Cl_2O_3$ [263,1], $Kp_{0,3}$: 115–117°C;

$[\alpha]_D^{20}$ = -37,1° (unverdünnt)

(-)-2-(2,6-Dichlorphenoxy)-propionsäure-N-(2-aminoethyl)-amid.

46,0 g (= 0,1748 Mol) (-)-2-(2,6-Dichlorphenoxy)-propionsäureethylester werden mit 212,0 g (= 3,527 Mol) 1,2-Diaminoethan 6 Stunden bei Raumtemperatur gerührt und ergeben nach Aufarbeitung 38,0 g (-)-2-(2,6-Dichlorphenoxy)-propionsäure-N-(2-aminoethyl)-amid, $C_{11}H_{14}Cl_2N_2O_2$ [277,2] als hochviskoses Öl;

$[\alpha]_D^{20}$ = –5,8° (c = 1/Ethanol)

(-)-2-[1-(2,6-Dichlorphenoxy)-ethyl]-1,3-diazacyclopenten-(2).

58,6 g (= 0,3089 Mol) Titantetrachlorid werden bei 0°C in einer Mischung aus 700 ml Chloroform abs. und 25 ml Tetrahydrofuran gelöst und mit 35,0 g (= 0,1263 Mol) (-)-2-(2,6-Dichlorphenoxy)-propionsäure-N-(2-aminoethyl)-amid versetzt. Anschließend läßt man sehr langsam bei 0°C unter Rühren eine Lösung von 74,0 g (= 0,6056 Mol) 4-Dimethylaminopyridin zutropfen und den Ansatz nach Beendigung der Zugabe noch 36 Stunden bei Raumtemperatur weiterrühren. Nach Aufarbeitung und säulenchromatographischer Reinigung (Kieselgel-Trockensäule, Elutionsmittel: Chloroform/Tetrahydrofuran 3:1) erhält man 21,0 g (-)-2-[1-(2,6-Dichlorphenoxy)-ethyl]-1,3-diazacyclopenten-(2), $C_{11}H_{12}Cl_2N_2O$ [259,1], Fp. 126-127°C;

$[\alpha]_D^{20}$ = –80,2° (c = 1/Ethanol).

(-)-2-[1-(2,6-Dichlorphenoxy)-ethyl]-1,3-diazacyclopenten-(2)hydrochlorid.

10,0 g (-)-2-[1-(2,6-Dichlorphenoxy)-ethyl]-1,3-diazacyclopenten-(2) werden in 40 ml Propanol-(2) gelöst und mit 40 ml einer gesättigten Lösung von Chlorwasserstoff in Propanol-(2) versetzt. Man erhält nach entsprechender Aufarbeitung 7,8 g (-)-2-[1-(2,6-Dichlorphenoxy)-ethyl]-1,3-diazacyclopenten-(2)-hydrochlorid, $C_{11}H_{12}Cl_2N_2O \cdot HCl$ [295,6], Fp. 229–230°C;

$[\alpha]_D^{20}$ = -33,2° (c = 1/Ethanol)

**Beispiel 2**

Synthese von (-)-2-[1-(2,6-Dichlorphenoxy)-ethyl]-1,3-diazacyclopenten-(2)-hydrochlorid

Die Ausgangssubstanz ist in Beispiel 1, Stufe 2, beschrieben.

(+)-2-(2,6-Dichlorphenoxy)-propionsäureamid.

In eine Lösung von 60 g (= 0,2281 Mol) (-)-2-(2,6-Dichlorphenoxy)-propionsäureethylester in 100 ml mit Ammoniak gesättigtem Ethanol wird

24 Stunden lang Ammoniak unter Rühren bei Raumtemperatur eingeleitet. Nach Aufarbeitung erhält man 41,0 g (+)-2-(2,6-Dichlorphenoxy)-propionsäureamid, $C_9H_9Cl_2NO_2$ [234,1], Fp. 193°C;

$[\alpha]_D^{20}$ = + 20,1° (c = 1/Aceton)

(-)-2-(2,6-Dichlorphenoxy)-propionitril.

Eine Lösung von 16,3 g (= 0,0859 Mol) Titantetrachlorid in 50 ml Chloroform abs. und 7 ml Tetrahydrofuran wird mit 10,0 g (= 0,0427 Mol) (+)-2-(2,6-Dichlorphenoxy)-propionsäureamid versetzt. Anschließend läßt man sehr langsam unter Rühren bei 0°C eine Lösung von 17,3 g (= 0,1709 Mol) 4-Methylmorpholin eintropfen und den Kolbeninhalt nach der Beendigung der Zugabe noch 24 Stunden bei 30°C weiterrühren. Nach Aufarbeitung erhält man 7,0 g (-)-2-(2,6-Dichlorphenoxy)-propionitril, $C_9H_7Cl_2NO$ [216,1], $Kp_{0,2}$: 85°C;

$[\alpha]_D^{20}$ = –76,6° (unverdünnt)

(-)-2-[1-(2,6-Dichlorphenoxy)-ethyl]-1,3-diazacyclopenten-(2).

20,0 g (= 0,0925 Mol) (-)-2-(2,6-Dichlorphenoxy)-propionitril werden bei 0°C mit 4,3 g (= 0,0925 Mol) Ethanol in 50 ml Chloroform abs. durch Einleiten von Chlorwasserstoff in das (-)-2-(2,6-Dichlorphenoxy)-propionimidsäureethylester-hydrochlorid überführt. Dieses wird nach dem Abziehen des Lösungsmittels im Vakuum bei 20°C anschließend in 20 ml Ethanol gelöst und mit 6,0 g (= 0,1 Mol) 1,2-Diaminoethan zum (-)-2-[1(2,6-Dichlorphenoxy) - ethyl] - 1,3 - diazacyclopenten-(2), $C_{11}H_{12}Cl_2N_2O$ [259,1] cyclisiert. Man erhält nach Aufarbeitung 16 g Produkt. Fp. 127-128°C;

$[\alpha]_D^{20}$ = -80,7° (c = 1/Ethanol).

(-)-2-[1-(2,6-Dichlorphenoxy)-ethyl]-1,3-diazacyclopenten-(2)-hydrochlorid.

4,0 g (-)-2-[1-(2,6-Dichlorphenoxy)-ethyl]-1,3-diazacyclopenten-(2) werden in 16 ml Propanol-(2) gelöst und mit 16 ml einer gesättigten Lösung von Chlorwasserstoff in Propanol-(2) versetzt. Man erhält 3,3 g (-)-2-[1-(2,6-Dichlorphenoxy)-ethyl]-1,3-diazacyclopenten-(2)-hydrochlorid, $C_{11}H_{12}Cl_2N_2O \cdot HCl$ [295,6], Fp. 228-229°C;

$[\alpha]_D^{20}$ = –33,4° (c = 1/Ethanol)

**Beispiel 3**

Gewinnung von (-)-2-[1-(2,6-Dichlorphenoxy)-ethyl]-1,3-diazacyclopent-(2)-en durch Racematspaltung von (±)-2-[1-(2,6-Dichlorphenoxy)-ethyl]-1,3-diazacyclopent-2-en mit (-)-Dibenzoylweinsäure.

10 g (±)-2-[1-(2,6-Dichlorphenoxy)-ethyl]-1,3-diazacyclopent-(2)-en werden bei Raumtemperatur in 350 ml Aceton gelöst, unter Rühren zu einer Lösung von 14,5 g (-)-Dibenzoylweinsäure $[\alpha]_D^{20}$ = –110° (c = 1,08/Ethanol 96 %) in 1500 ml Aceton gegeben und ohne Rühren 24 Stunden bei 21°C stehen gelassen. Das angefallene Salz (12,5 g) liegt als (-)-Dibenzoyltartrat der angereicherten (-)-Base vor und besitzt den Drehwert $[\alpha]_D^{20}$ = –64° (c = 1/Ethanol). Aus diesem Salz erhält man nach Neutralisation mit gesättigter Natriumcarbonatlösung und Extraktion mit Dichlormethan 6,5 g Base, die nach Lösen in 520 ml Aceton erneut mit einer Lösung von 9,4 g (-)-Dibenzoylweinsäure in 1950 ml Aceton versetzt wird. Nach 24 Stunden erhält man 9,6 g des (-)-Dibenzoyltartrats der jetzt bereits hoch angereicherten (-)-Base [$[\alpha]_D^{20}$ = –70,9° (c = 1/ Ethanol)]. Die weitere Reinigung erfolgt durch Umkristallisation von 9,5 g dieses Salzes aus 180 ml 60°C heißem Ethanol. Nach 24 Stunden (21°C) erhält man 5,9 g (-)-Dibenzoyltartrat der (-)-Base mit dem Drehwert $[\alpha]_D^{20}$ = –72,4° (c = 1/Ethanol). Die zweite Umkristallisation von 5,8 g die-ses Salzes aus 90 ml 60°C heißem Ethanol ergibt nach 24 Stunden 3,8 g (-)-2-[1-(2,6-Dichlorphenoxy)-ethyl]-1,3-diazacyclopenten-(2)-(-)-di-benzoyltartrat mit dem Drehwert $[\alpha]_D^{20}$ = –76,9° (c = 1/Ethanol). Aus 3,7 g des letzten Salzes werden mit 20 ml gesättigter Natriumcarbonatlösung und 50 ml Dichlormethan 1,8 g reine (-)-Base freigesetzt, die in 3 ml Propanol-(2) gelöst und mit 3 ml einer gesättigten Lösung von Chlorwasserstoff in Propanol-(2) versetzt und in das Hyd-rochlorid überführt wird. Man erhält 1,4 g (-)-2-[1(2,6-Dichlorphenoxy)-ethyl]-1,3-diazacyclopenten-(2)-hydrochlorid, $C_{11}H_{12}Cl_2N_2O \cdot HCl$ [295,6], Fp. 228-229°C;

$[\alpha]_D^{20}$ = –33,7° (c = 1/Ethanol)

**Patentanspruch**

Verfahren zur Herstellung von (-)-2-[1-(2,6-Dichlorphenoxy)-ethyl]-1,3-diazacyclopent-2-en und seiner pharmazeutisch verträglichen Säureadditionssalze, dadurch gekennzeichnet, daß man (-)-2-Hydroxypropionsäure-$C_{1-4}$-Alkylester mit Thionylchlorid in Gegenwart katalytischer Mengen von Dimethylformamid unter Konfigurationsumkehr zum (+)-2-Chlorpropionsäure-$C_{1-4}$-Alkylester umsetzt, diesen mit 2,6-Dichlorphenol unter erneuter Konfigurationsumkehr in einem geeigneten organischen Lösungsmittel in Gegenwart von Hilfsbasen im Temperaturbereich von 60-150°C in den (-)-2-(2,6-Dichlorphenoxy)-propionsäure-$C_{1-4}$-Alkylester überführt und diesen nach an sich bekannten Methoden in das (-)-1,3-Diazacyclopenten-Derivat umwandelt und, gegebenenfalls, diese Base in pharmazeutisch verträgliche Säureadditionssalze umwandelt.

**Claim**

Process for the production of (-)-2-[1-(2,6-dichlorophenoxy)-ethyl]-1,3-diazacyclopent-2-ene and the pharmaceutically acceptable acid addition salts thereof, characterised in that a (-)-2-hydroxypropionic acid-$C_{1-4}$ alkylester is reacted with thionylchloride in the presence of catalytic quantities of dimethylformamide with configuration reversal to produce a (+)-2-chloropropionic acid-$C_{1-4}$ alkyl ester which is converted into (-)-2-(2,6-dichlorophenoxy)-propionic acid-$C_{1-4}$

alkyl ester with a further configuration reversal, by etherification with 2,6-dichlorophenol in the presence of a base at a temperature in the range from 60 to 150°C in a suitable organic solvent, this alkyl ester being used for the production of the (-)-1,3-diazacyclopentene derivative according to known methods, and, possibly, the base being converted into pharmaceutically acceptable acid addition salts thereof.

**Revendiaction**

Procédé de préparation du (-)-2-[1-(2,6-dichlorophénoxy)-éthyl]-1,3-diazacyclopenta-2-ène et de ses sels formés par addition avec des acides acceptables pour l'usage pharmaceutique, caractérisé en ce que l'on convertit un (-)-2-hydroxypropionate d'alkyle en C 1–C 4 à l'aide du chlorure de thionyle en présence de quantités catalytiques de diméthylformamide, avec inversion de la configuration, en un (+)-2-chloropropionate d'alkyle en C 1–C 4, on convertit ce dernier, par le 2,6-dichlorophénol, avec nouvelle inversion de la configuration, dans un solvant organique approprié, en présence de bases auxiliaires, dans l'intervalle de température de 60 à 150°C, en le (-)-2-(2,6-dichlorophénoxy)-propionate d'alkyle en C 1–C 4 qu'on convertit lui-même selon des modes opératoires connus en soi, en le dérivé de (-)-1,3-diazacyclopentène qu'on convertit le cas échéant en un sel par addition avec un acide acceptable pour l'usage pharmaceutique.